# EUROPEAN PATENT APPLICATION

(11) **EP 4 317 177 A1**
(43) Date of publication of application: **07.02.2024**
(21) Application number: 22778407.1
(22) Date of filing: 23.02.2022
(51) Int. Cl.: C07K 14/165, C12N 15/50, C12N 15/85, C12N 5/10, A61K 39/215, A61P 31/14, A61P 11/00

(54) **NOVEL CORONAVIRUS S-RBD TRIMERIC PROTEIN VACCINE, PREPARATION METHOD THEREFOR, AND APPLICATION THEREOF**

(30) Priority: 31.03.2021 CN 202110348881
(71) Applicant: National Vaccine and Serum Institute (NVSI), Beijing 101111 (CN)
(72) Inventor: LI, Qiming, Beijing 101111 (CN); LIANG, Yu, Beijing 101111 (CN); SU, Jiguo, Beijing 101111 (CN); ZHANG, Jing, Beijing 101111 (CN); JIN, Yuqin, Beijing 101111 (CN); HOU, JunWei, Beijing 101111 (CN); DU, Lifang, Beijing 101111 (CN); HAN, Zibo, Beijing 101111 (CN); ZHANG, Xuefeng, Beijing 101111 (CN); SHAO, Shuai, Beijing 101111 (CN); ZHANG, Hao, Beijing 101111 (CN); TANG, Fang, Beijing 101111 (CN); LIU, Zhaoming, Beijing 101111 (CN); HOU, Yanan, Beijing 101111 (CN); CHEN, Shi, Beijing 101111 (CN); LEI, Zehua, Beijing 101111 (CN); MA, Zhijing, Beijing 101111 (CN); ZHENG, Fan, Beijing 101111 (CN); LIU, Ning, Beijing 101111 (CN)
(74) Representative: Cabinet Chaillot
(86) International application number: PCT/CN2022/077527
(87) International publication number: WO 2022/206222

(57) **Abstract**

Disclosed is a novel coronavirus S-RBD trimeric protein. The trimeric protein is composed of amino acid fragments of positions 319-537 in an RBD region of a novel coronavirus S protein in a trimeric form. A vaccine prepared by the present invention uses the S-RBD trimeric protein as an antigen; once an adjuvant is added, the body can be immunized and high-titer protective neutralizing antibodies against the novel coronavirus can be produced. The vaccine can be used for the treatment and/or prevention of novel coronavirus (SARS-CoV-2) infections and/or novel coronavirus diseases.

## Description

### CROSS-REFERENCE

The present application claims the priority of a Chinese patent application entitled "S-RBD TRIMER PROTEIN VACCINE FOR NOVEL CORONAVIRUS AND PREPARATION METHOD AND APPLICATION THEREFOR" submitted to the Chinese Patent Office on Wednesday, March 31, 2021, with the application number of 202110348881.6, and the entire content of which is incorporated in the present application by reference.

### BACKGROUND OF THE INVENTION

### 1. Technical Field

The present invention relates to the field of biological medicines, and in particular to an S-RBD trimer protein vaccine for a novel coronavirus and a preparation method and an application therefor.

### 2. Description of Related Art

In 2020, a coronavirus disease (COVID-19) caused by severe acute respiratory syndrome coronavirus 2 (SARS-CoV-2) has been pandemic globally. Its rapid propagation speed, wide spreading regions and a large number of infected persons are very rare in decades. The novel coronavirus pneumonia is worse than SARS and MERS viruses in severity. So far, the novel coronavirus has spread to more than 200 countries and regions around the world. Data from World Health Organization (World Health Organizatio) shows that by February 23, 2021, a total number of confirmed cases around the world exceeded 110 million, and a death number exceeded 2.46 million. Although measures of mask wearing and quarantining can prevent virus infection, the novel coronavirus will severely affect global economic development and daily life of people; and only a vaccine is an optimal means for ending the epidemic. Some experts said that the novel coronavirus would coexist with humans for a long time, and had a possibility of repeated outbreak. However, as an ultimate weapon for winning the battle against the epidemic, the vaccine will exist for a long time as well. Therefore, businesses around the world participate in research and development of the vaccines against the novel coronavirus, and there have been more than 100 kinds of vaccines against the novel coronavirus in different development stages all over the world. It can be seen therefrom that the vaccine against the novel coronavirus has a wide developing prospect.

SARS-CoV-2 belongs to the Nidovirales of the family Coronaviridae, subfamily Positive coronavirus, genus Betacoronavirus, subgenus Sarbecovirus, species SARS-like virus, is a single-stranded positive-sense RNA virus, is enveloped, and has a genome with a full length of about 29.9 kb; the vast majority of the sequence encodes non-structural proteins and participates to functions of virus replication, translation and the like, and a small part of the sequence encodes structural proteins, such as a spike protein (S), a membrane protein (an M protein), an envelope protein (an E protein) and a nucleo protein (an N protein); and in addition, there are a plurality of accessory proteins: 3a, 3b, p6, 7a, 7b, 8b, 9b and orf14, and these proteins all participate to virus assembly. The S, M and E proteins form a virus cyst membrane which is a major surface antigen for a virus inducing an immune response. The S protein is a transmembrane glycoprotein, has a molecular weight about 150 kDa, and forms a prominent homotrimer on the surface of the virus. The S protein consists of two functional subunits, and is cleaved at a boundary (an S1/S2 cleavage site) between the S1 subunit and the S2 subunit; and the two subunits keep non-covalent binding in conformation before fusion. The S2 subunit also consists of a plurality of structural domains, and has a major function of mediating fusion of the virus to the host cell. There are four different structural domains of the distal S1 subunit in structure: NTD, RBD, CTD1 and CTD2, where RBD is a receptor binding structural domain, and is mainly responsible for binding to a receptor angiotensin converting enzyme 2 (ACE2) on the surface of the host cell, thereby mediating the virus to infect the host cell. Therefore, the S protein and the RBD are both major targets for current research and development of genetic engineering vaccines.

So far, there have been 7 kinds of approved and marketed vaccines globally in total: BNT162b2 and mRNA-1273 under emergency use authorization (EUA) approved by U.S.; AZD1222 under emergency use authorization (EUA) approved by England; 3 kinds of inactivated vaccines against the novel coronavirus, conditionally marketed by China National Vaccine & Serum Institute (Beijing Company and Wuhan Company) and Sinovac Biotech Co., Ltd, an adenovirus vector vaccine from CanSino Biologics Inc., and a recombinant protein vaccine from Zhifei Biologics Company; a domestic inactivated vaccine under emergency use authorization (EUA) approved by India; and "satellite V" approved and marketed by Russia. In addition, there are further dozens of vaccines entering different stages of clinical research. Research and development of recombinant vaccines using the genetic engineering technology have been widely proved due to its high safety and effectiveness; and in addition, current novel coronavirus variant strains emerge continuously with a proportion continuously raised. The protecting effect of existing vaccines and neutralizing antibodies to specific variant strains is greatly weakened, which sparks worries on trend of the novel coronavirus epidemic and the effectiveness of the vaccines and drugs from all walks of life.

Therefore, it is of great urgency to develop a recombinant vaccine capable of producing a high-titer protective neutralizing antibody for the novel coronavirus.

### BRIEF SUMMARY OF THE INVENTION

In one aspect of the present invention, an S-RBD trimer protein for a novel coronavirus is provided, aiming at the defects in the prior art of lack of a recombinant vaccine capable of producing a high-titer protective neutralizing antibody for the novel coronavirus.

The technical solution of the present invention is as follows:
an S-RBD trimer protein for a novel coronavirus is characterized by being composed of amino acid fragments at positions 319-537 in an RBD domain of an S protein of the novel coronavirus in a trimer form.

In the present invention, based on the structural features of the S-RBD domain of the novel coronavirus, a computational biological method is used to design a brand new fusion protein which includes three RBD structural domains, and the trimer form with stable antigen conformation may be formed in the case without introducing any exogenous linking arm or other irrelevant components, thereby achieving S-RBD protein trimerization. After the S-RBD trimer protein is subjected to recombined expression and purified using the genetic engineering technology, the S-RBD trimer protein is mixed with the adjuvant to prepare a vaccine; and the high-titer protective neutralizing antibody for the novel coronavirus may be produced by immunizing an animal with the vaccine according to a certain dosage and a number of doses, and is used for treating and/or treating novel coronavirus (SARS-CoV-2) infection and/or a novel coronavirus disease (COVID-19). In addition, due to their definite functions and a clear structure, the RBD domains are responsible for identifying an ACE domain of a receptor cell; and meanwhile, an antibody produced for the RBD is definite in function, so that the body is avoided from being induced to generate antibody dependent enhancement (ADE) to the maximum.

The trimer protein in the present invention may be formed by self-assembly of three polypeptide subunits including a same sequence. In the embodiments of the present invention, a suitable linker or spacer may be included between the above three same sequences, may be an oligopeptide or a polypeptide and may have the effect of improving the flexibility.

However, preferably, in one embodiment of the present invention, a primary structure of the trimer protein is formed by linking three of the amino acid fragments in an order from an N terminal to a C terminal.

More preferably, in one embodiment of the present invention, the fusion protein includes an amino acid sequence shown as SEQ ID No. 1 or a sequence having a homology of 95% or higher with SEQ ID No. 1.

In the above embodiments, the trimer protein of the present invention may form the trimer form with stable antigen conformation in the case without introducing any exogenous linking arm or other irrelevant components.

The sequence having the homology of 95% or higher with SEQ ID No. 1 refers to an amino acid sequence having 95%, 96%, 97%, 98% or 99% identity to the amino acid sequence of the fusion protein. Those skilled in the art may make random or engineered point mutation on the amino acid sequence of the fusion protein in this specification in a suitable manner, an objective of which may be, for example, to obtain better affinity and/or dissociation property; however, these amino acid sequences after mutation are all within the scope of the present invention.

In another aspect of the present invention, a fusion protein is provided, including the S-RBD trimer protein for the novel coronavirus.

Preferably, in the embodiments of the present invention, the fusion protein further includes one or more of a signal peptide, a tag or an immune-enhancing peptide. The signal peptide may have the effect of more facilitating expression of the protein. The tag may be, for example, a Flag tag, an enhanced green fluorescence protein (eGFP), a glutathione S-transferase (GST) and the like, and may have the effect of being used for detection, purification, separation and the like. The above functional sequences may be arbitrarily combined for use.

In another aspect of the present invention, a nucleic acid molecule is provided, including a nucleotide sequence encoding the S-RBD trimer protein for the novel coronavirus or the fusion protein.

Preferably, in one embodiment of the present invention, the inventor optimizes a codon of the trimer protein, and the obtained nucleotide sequence is shown as SEQ ID No. 2 or an obtained sequence has a homology of 95% or higher with SEQ ID No. 2.

The sequence having the homology of 95% or higher with SEQ ID No. 2 refers to the nucleotide sequence having 95%, 96%, 97%, 98% or 99% identity to the nucleotide sequence.

For a preparation method for the above nucleic acid molecule, the nucleic acid molecule may be prepared by chemical synthesis, PCR amplification and other known technologies based on the above nucleotide sequence. Generally, the codon encoding the amino acids of the above structural domains may be optimized, so as to optimize expression of the codon in a host cell. Information about the above base sequence may be retrieved from known literatures, NCBI (https://www.ncbi.nlm.nih.gov/) or other databases.

In another aspect of the present invention, a vector is provided, including the nucleic acid molecule.

In the present invention, the vector may be a straight-chain vector and also a circular vector. The vector may be a non-viral vector such as a plasmid, a viral vector and also a vector using a transposon. The vector may include a promoter, a terminator or other regulatory sequences, and a drug resistance gene, a reporter gene or other marker sequence.

Preferably, in one embodiment of the present invention, the vector is an expression vector for the nucleic acid molecule in the present invention.

In another aspect of the present invention, a host cell is provided, including the above nucleic acid molecule or the above vector.

Preferably, in the embodiments of the present invention, the host cell is Escherichia coli, a yeast cell, an insect cell or a mammalian cell.

More preferably, in one embodiment of the present invention, the host cell is a Chinese hamster ovary (CHO) cell.

In another aspect of the present invention, a preparation method for the S-RBD trimer protein for the novel coronavirus or the fusion protein is provided, including the following steps:
step A): preparing the nucleic acid molecule, constructing the expression vector, and transforming or transfecting the expression vector into the host cell;
step B): performing, by using a product in step A), protein expression; and
step C): obtaining, by purifying an expression product obtained in step B), the S-RBD trimer protein for the novel coronavirus or the fusion protein.

In step A), the nucleic acid molecule includes a nucleotide sequence encoding the S-RBD trimer protein for the novel coronavirus or the fusion protein.

Preferably, in one embodiment of the present invention, the nucleic acid molecule includes a nucleotide sequence shown as SEQ ID No. 2 or a sequence having a homology of 95% or higher with SEQ ID No. 2.

The nucleic acid molecule may be prepared using an arbitrary suitable molecular biological method according to the nucleotide sequence in this specification.

In constructing the expression vector in step A), the nucleotide sequence may be constructed in the corresponding expression vector of the host cell using an arbitrary suitable method.

Then, the expression vector is transformed or transfected into the host cell. Preferably, in one embodiment of the present invention, the inventor transfects the expression vector for the CHO cell into a 293FT cell or the CHO cell for construction of a recombinant cell line after the expression vector for the CHO cell is constructed.

For protein expression in step B), a recombinant protein may be expressed according to different used expression systems. Further, in one embodiment of the present invention, the inventor performs screening with a limited dilution method, to obtain a cell line capable of stable secretory expression of the S-RBD trimer protein or the fusion protein.

Purifying in step C) may be an arbitrary suitable method, for example, a salting out method, a precipitation method, dialysis or ultrafiltration, molecular sieve chromatography, ion exchange chromatography, hydrophobic chromatography, affinity chromatography and the like. Preferably, in one embodiment of the present invention, the S-RBD trimer protein or the fusion protein is purified using ion exchange chromatography and hydrophobic chromatography.

Certainly, according to the prior art, before step of purifying, the preparation method should further include a collection process of target proteins, for example, collection of a cell culture supernatant rich in the target proteins. The process of breaking the host cell after the target proteins are expressed may uses, for example, ultrasonic disruption, breaking with repeated freeze thawing, a chemical treatment method or other arbitrary suitable breaking methods. The collection process of the host cell should also be understood as being within the scope of the purifying.

In another aspect of the present invention, a use of the S-RBD trimer protein for the novel coronavirus, the fusion protein, the nucleic acid molecule, the vector or the host cell in preparing a drug for treating and/or preparing novel coronavirus infection and/or a disease caused by the novel coronavirus is provided.

The disease caused by the novel coronavirus, is preferably, novel coronavirus pneumonia (COVID-19).

In another aspect of the present invention, a vaccine is provided, including the S-RBD trimer protein for the novel coronavirus or the fusion protein, and the adjuvant.

In the embodiments of the present invention, the vaccine is a recombinant protein vaccine (also called genetic engineering subunit vaccine). Further, in some another embodiments of the present invention, the vaccine may further be a genetic engineering vector vaccine or a nucleic acid vaccine; and the above vaccines include the nucleotide sequence in this specification.

The vaccine of the present invention may include an arbitrary suitable adjuvant. However, preferably, in the embodiments of the present invention, the adjuvant is aluminium hydroxide, aluminium phosphate, MF59 or CpG. More preferably, the adjuvant is the aluminium hydroxide.

In another aspect of the present invention, a preparation method for the vaccine is provided, where the S-RBD trimer protein for the novel coronavirus or the fusion protein obtained by purification is mixed with the adjuvant.

In another aspect of the present invention, a use of the vaccine in treating and/or preparing novel coronavirus infection and/or a disease caused by the novel coronavirus is provided.

The disease caused by the novel coronavirus, is preferably, novel coronavirus pneumonia (COVID-19).

In another aspect of the present invention, a drug composition is provided, the drug composition including the vaccine and a pharmaceutically acceptable vector.

The pharmaceutically acceptable vector may be an arbitrary pharmaceutically acceptable additive, for example, normal saline, a cell culture medium, glucose, water for injection, glycerol, amino acid, a combination thereof, a stabilizing agent, a surfactant, a preservative, an isotonic agent and the like.

The drug composition of the present invention may further be used in combination with other drugs for treating and/or preventing novel coronavirus infection and/or the disease caused by the novel coronavirus with an effective and safe dosage.

In another aspect of the present invention, a method of eliciting an immune response of a subject to the novel coronavirus or treating the novel coronavirus infection of the subject is provided, in which an effective dosage of the vaccine or the drug composition is applied to the subject.

The subject may be a human or other animals.

Application may be intramuscular injection, intraperitoneal injection or subcutaneous injection.

### Beneficial effects of the present invention:

A body is immunized with a vaccine prepared in the present invention taking the S-RBD trimer protein as an antigen and supplemented by an adjuvant, and then a high-titer protective neutralizing antibody for the novel coronavirus may be produced and may be used for treating and/or preventing novel coronavirus (SARS-CoV-2) infection and/or a novel coronavirus disease.

### BRIEF DESCRIPTION OF THE SEVERAL VIEWS OF THE DRAWINGS

FIG. 1 is structure analysis diagram of an S protein for a novel coronavirus in example 1 of the present invention, where A is an S protein monomer structure (drawn using a UCSF Chimera software based on a coordinate file with a PDB code being 6zgg); an S 1 structure unit includes NTD, RBD, SD1 and SD2 structural domains; and B is a composite structure of a RBD and an ACE2 receptor (drawn using the UCSF Chimera software based on a coordinate file with the PDB code being 6m0j);
FIG. 2 is a design diagram of an S-RBD trimer protein in example 1 of the present invention, where A is an S-RBD monomer protein structure; and B is an S-RBD trimer protein structure;
FIG. 3 is a sodium dodecyl sulphate-polyacrylamide gel electrophoresis (SDS-PAGE) diagram of an S-RBD trimer protein purified in example 2 of the present invention, where lanes 1-5 are S-RBD trimer proteins obtained by purification; a lane M1 is a protein marker (with a molecular weight standard being kDa: 250, 150, 100, 70, 50, 40, 30, 20, 15, 10 and 5); and a lane M2 is a protein marker (with a molecular weight standard being kDa: 250, 130, 100, 70, 55, 35, 25, 15 and 10);
FIG. 4 is a Western-blot identification diagram of an S-RBD trimer protein purified in example 2 of the present invention, where lanes 1-5 are S-RBD trimer proteins obtained by purification; and a lane M2 is a protein marker;
FIG. 5 is a transmission electron micrograph of an S-RBD trimer protein purified in example 2 of the present invention;
FIG. 6 is a binding curve diagram of an S-RBD trimer protein purified in example 3 of the present invention and an MM43 neutralizing monoclonal antibody;
FIG. 7 is a curve diagram of an S-RBD trimer protein purified in example 3 of the present invention binding to an MM57 neutralizing monoclonal antibody;
FIG. 8 is an SPR detection result diagram of an S-RBD trimer protein purified in example 4 of the present invention and an ACE2 protein.
FIG. 9 is a detection result diagram of a titer of serum-specific IgG after Wistar rats are immunized with different dosages of a recombinant novel coronavirus vaccine in example 6 of the present invention;
FIG. 10 is a detection result diagram of a titer of a serum neutralizing antibody for pseudovirus after Wistar rats are immunized with different dosages of a recombinant novel coronavirus vaccine in example 6 of the present invention;
FIG. 11 is a detection result diagram of a titer of a serum neutralizing antibody for a wild virus after Wistar rats are immunized with different dosages of a recombinant novel coronavirus vaccine in example 6 of the present invention; FIG. 12 is an SDS-PAGE diagram of a dimer protein and a trimer protein in comparative example 1 of the present invention, where a lane 1 is a trimer protein obtained by purification; a lane 2 is a dimer protein obtained by purification; and a lane M2 is a protein marker (with a molecular weight standard being kDa: 250, 130, 100, 70, 55, 35, 25, 15 and 10);
FIG. 13 is a detection result diagram of a titer of serum-specific IgG in comparative example 1 of the present invention;
FIG. 14 is a detection result diagram of a titer of a serum neutralizing antibody for a pseudovirus in comparative example 1 of the present invention; and
FIG. 15 is a detection result diagram of a titer of a serum neutralizing antibody for a wild virus in comparative example 1 of the present invention.

### SEQUENCE DESCRIPTION

SEQ ID No. 1 is an amino acid sequence of an S-RBD trimer protein for a novel coronavirus in examples of the present invention;
SEQ ID No. 2 is a nucleotide sequence of an S-RBD trimer protein for a novel coronavirus in examples of the present invention;
SEQ ID No. 3 is an amino acid sequence of an S-RBD dimer protein for a novel coronavirus in comparative examples of the present invention; and
SEQ ID No. 4 is a nucleotide sequence of an S-RBD dimer protein for a novel coronavirus in examples of the present invention.

### SEQUENCE TABLE

| Number | Sequence |
|---|---|
| SEQ ID No. 1 | MRVQPTESIVRFPNITNLCPFGEVFNATRFASVYAWNRKRISNCVAD YSVLYNSASFSTFKCYGVSPTKLNDLCFTNVYADSFVIRGDEVRQIA PGQTGKIADYNYKLPDDFTGCVIAWNSNNLDSKVGGNYNYLYRLF RKSNLKPFERDISTEIYQAGSTPCNGVEGFNCYFPLQSYGFQPTNGV GYQPYRVVVLSFELLHAPATVCGPKKSTNLVKNKRVQPTESIVRFPN ITNLCPFGEVFNATRFASVYAWNRKRISNCVADYSVLYNSASFSTFK CYGVSPTKLNDLCFTNVYADSFVIRGDEVRQIAPGQTGKIADYNYK LPDDFTGCVIAWNSNNLDSKVGGNYNYLYRLFRKSNLKPFERDIST EIYQAGSTPCNGVEGFNCYFPLQSYGFQPTNGVGYQPYRVVVLSFE LLHAPATVCGPKKSTNLVKNKRVQPTESIVRFPNITNLCPFGEVFNAT RFASVYAWNRKRISNCVADYSVLYNSASFSTFKCYGVSPTKLNDLC FTNVYADSFVIRGDEVRQIAPGQTGKIADYNYKLPDDFTGCVIAWN SNNLDSKVGGNYNYLYRLFRKSNLKPFERDISTEIYQAGSTPCNGVE GFNCYFPLQSYGFQPTNGVGYQPYRVVVLSFELLHAPATVCGPKKS TNLVKNK |
| SEQ ID NO.2 | ATGAGAGTGCAGCCCACAGAGTCAATTGTGAGATTCCCCAATATC ACTAACCTGTGCCCCTTTGGGGAGGTGTTCAATGCCACAAGATTT GCTTCTGTGTATGCCTGGAATAGAAAGAGAATCAGTAACTGTGTT GCTGACTACTCTGTGCTGTACAATTCTGCCTCTTTCAGCACATTCA AATGTTATGGGGTTAGCCCCACCAAACTAAATGACCTGTGCTTCA CCAATGTGTATGCTGACAGCTTTGTGATCAGAGGTGATGAAGTGA GACAAATTGCCCCTGGGCAGACTGGCAAGATAGCTGACTACAATT ACAAGCTGCCTGATGACTTTACTGGCTGTGTGATTGCCTGGAATA GCAATAACCTGGACAGCAAGGTGGGGGGCAACTATAACTACCTAT ACAGATTGTTTAGAAAGTCCAATTTGAAACCCTTTGAGAGAGATA TCAGCACTGAGATCTATCAAGCTGGCAGCACCCCCTGTAATGGGG TGGAGGGGTTCAACTGCTATTTCCCCTTACAGAGCTATGGCTTTC AGCCCACCAATGGGGTGGGCTATCAGCCCTACAGAGTGGTGGTG CTGTCTTTTGAACTACTTCATGCCCCTGCCACAGTGTGTGGCCCC AAAAAAAGCACAAACCTGGTGAAAAACAAGAGAGTGCAGCCAA |
| | CAGAGAGCATTGTGAGATTCCCTAACATAACCAATCTGTGTCCCT TTGGTGAGGTTTTTAATGCTACTAGATTTGCCTCTGTGTATGCCTG GAACAGAAAAAGAATCTCAAACTGTGTGGCTGACTATTCAGTCC TCTACAACTCAGCTAGCTTTAGCACCTTCAAATGCTATGGTGTCA GTCCCACCAAACTGAATGACCTTTGTTTCACCAATGTCTATGCTG ACAGCTTTGTGATCAGAGGGGATGAAGTGAGACAAATAGCCCCT GGGCAGACTGGCAAGATAGCTGATTACAACTATAAACTGCCTGAT GATTTCACTGGCTGTGTCATTGCCTGGAACTCAAATAATCTAGAC AGCAAGGTAGGTGGCAATTATAACTACCTCTATAGGCTGTTTAGG AAAAGTAACCTGAAACCCTTTGAGAGAGACATTAGCACAGAGAT TTATCAAGCTGGCTCAACACCCTGTAATGGTGTGGAGGGCTTTAA CTGCTACTTCCCCCTGCAGAGCTATGGCTTTCAGCCAACCAATGG AGTGGGCTATCAGCCCTATAGAGTAGTTGTGCTGAGCTTTGAGCT GCTGCATGCCCCTGCCACAGTGTGTGGCCCTAAGAAAAGCACCA ACTTGGTCAAAAATAAGAGAGTACAACCCACAGAGTCCATTGTG AGATTTCCCAACATCACTAATTTGTGTCCTTTTGGGGAGGTCTTCA ATGCCACAAGATTTGCCTCTGTGTATGCCTGGAACAGAAAGAGAA TCAGCAACTGTGTGGCTGACTACTCTGTGCTGTACAACTCTGCCT CCTTCAGCACCTTCAAGTGCTATGGGGTGAGCCCCACCAAGCTC AATGACTTATGCTTCACCAATGTGTATGCTGACAGCTTTGTGATTA GAGGGGATGAGGTCAGACAGATTGCCCCTGGCCAAACTGGAAA AATTGCTGACTACAACTACAAACTGCCTGATGACTTTACTGGCTG TGTGATTGCCTGGAACTCCAACAACCTTGATAGCAAGGTTGGGG GGAATTATAACTATCTGTACAGACTATTCAGAAAGAGCAACCTGA AACCCTTTGAGAGAGACATCTCAACAGAAATATATCAAGCTGGCT CCACTCCATGCAATGGGGTGGAGGGATTCAATTGTTACTTCCCCC TGCAGTCATATGGCTTTCAACCCACCAATGGGGTGGGCTACCAAC CTTACAGAGTGGTAGTACTTTCCTTTGAGCTACTTCATGCCCCTGC CACAGTGTGTGGCCCCAAGAAAAGCACCAATCTGGTAAAGAATA AGTGA |
| SEQ ID NO.3 | MRVQPTESIVRFPNITNLCPFGEVFNATRFASVYAWNRKRISNCVAD YSVLYNSASFSTFKCYGVSPTKLNDLCFTNVYADSFVIRGDEVRQIA PGQTGKIADYNYKLPDDFTGCVIAWNSNNLDSKVGGNYNYLYRLF |
| | RKSNLKPFERDISTEIYQAGSTPCNGVEGFNCYFPLQSYGFQPTNGV GYQPYRVVVLSFELLHAPATVCGPKKSTNLVKNKRVQPTESIVRFPN ITNLCPFGEVFNATRFASVYAWNRKRISNCVADYSVLYNSASFSTFK CYGVSPTKLNDLCFTNVYADSFVIRGDEVRQIAPGQTGKIADYNYK LPDDFTGCVIAWNSNNLDSKVGGNYNYLYRLFRKSNLKPFERDIST EIYQAGSTPCNGVEGFNCYFPLQSYGFQPTNGVGYQPYRVVVLSFE LLHAPATVCGPKKSTNLVKNK |
| SEQ ID NO.4 | ATGAGAGTGCAGCCCACAGAGTCAATTGTGAGATTCCCCAATATC ACTAACCTGTGCCCCTTTGGGGAGGTGTTCAATGCCACAAGATTT GCTTCTGTGTATGCCTGGAATAGAAAGAGAATCAGTAACTGTGTT GCTGACTACTCTGTGCTGTACAATTCTGCCTCTTTCAGCACATTCA AATGTTATGGGGTTAGCCCCACCAAACTAAATGACCTGTGCTTCA CCAATGTGTATGCTGACAGCTTTGTGATCAGAGGTGATGAAGTGA GACAAATTGCCCCTGGGCAGACTGGCAAGATAGCTGACTACAATT ACAAGCTGCCTGATGACTTTACTGGCTGTGTGATTGCCTGGAATA GCAATAACCTGGACAGCAAGGTGGGGGGCAACTATAACTACCTAT ACAGATTGTTTAGAAAGTCCAATTTGAAACCCTTTGAGAGAGATA TCAGCACTGAGATCTATCAAGCTGGCAGCACCCCCTGTAATGGGG TGGAGGGGTTCAACTGCTATTTCCCCTTACAGAGCTATGGCTTTC AGCCCACCAATGGGGTGGGCTATCAGCCCTACAGAGTGGTGGTG CTGTCTTTTGAACTACTTCATGCCCCTGCCACAGTGTGTGGCCCC AAAAAAAGCACAAACCTGGTGAAAAACAAGAGAGTGCAGCCAA CAGAGAGCATTGTGAGATTCCCTAACATAACCAATCTGTGTCCCT TTGGTGAGGTTTTTAATGCTACTAGATTTGCCTCTGTGTATGCCTG GAACAGAAAAAGAATCTCAAACTGTGTGGCTGACTATTCAGTCC TCTACAACTCAGCTAGCTTTAGCACCTTCAAATGCTATGGTGTCA GTCCCACCAAACTGAATGACCTTTGTTTCACCAATGTCTATGCTG ACAGCTTTGTGATCAGAGGGGATGAAGTGAGACAAATAGCCCCT GGGCAGACTGGCAAGATAGCTGATTACAACTATAAACTGCCTGAT GATTTCACTGGCTGTGTCATTGCCTGGAACTCAAATAATCTAGAC AGCAAGGTAGGTGGCAATTATAACTACCTCTATAGGCTGTTTAGG AAAAGTAACCTGAAACCCTTTGAGAGAGACATTAGCACAGAGAT TTATCAAGCTGGCTCAACACCCTGTAATGGTGTGGAGGGCTTTAA |
| | CTGCTACTTCCCCCTGCAGAGCTATGGCTTTCAGCCAACCAATGG AGTGGGCTATCAGCCCTATAGAGTAGTTGTGCTGAGCTTTGAGCT GCTGCATGCCCCTGCCACAGTGTGTGGCCCTAAGAAAAGCACCA ACTTGGTCAAAAATAAGTGA |

### DETAILED DESCRIPTION OF THE INVENTION

The present invention discloses an S-RBD trimer protein vaccine for a novel coronavirus and a preparation method and an application therefor. Those skilled in the art may make a proper improvement on process parameters for implementation with reference to the content of this specification. Of particular note, all similar substitutes and modifications are apparent to one of skill in the art and are considered to be included within the present invention; and related persons can obviously make a modification or a proper change and combination on the content of this paper without departing from the content, the spirit and the scope of the present invention, so as to implement and apply the technology of the present invention.

In the present invention, unless otherwise specified, scientific and technical terms used herein have the meaning as commonly understood by those skilled in the art. The following makes interpretation for a part of the terms in the present invention.

The term "novel coronavirus", i.e. SARS-CoV-2, belongs to the Nidovirales of the family Coronaviridae, subfamily Positive coronavirus, genus Betacoronavirus, subgenus Sarbecovirus, species SARS-like virus, is a single-stranded positive-sense RNA virus, is enveloped, and has a genome with a full length of about 29.9 kb; the vast majority of the sequence encodes non-structural proteins and participates to functions of virus replication, translation and the like, and a small part of the sequence encodes structural proteins, such as a spike protein (S), a membrane protein (an M protein), an envelope protein (an E protein) and a nucleo protein (an N protein); and in addition, there are a plurality of accessory proteins: 3a, 3b, p6, 7a, 7b, 8b, 9b and orf14, and these proteins all participate to virus assembly. The S, M and E proteins form a virus cyst membrane which is a major surface antigen for a virus inducing an immune response. The S protein is a transmembrane glycoprotein, has a molecular weight about 150 kDa, and forms a prominent homotrimer on the surface of the virus. The S protein consists of two functional subunits, and is cleaved at a boundary (an S1/S2 cleavage site) between the S 1 subunit and the S2 subunit; and the two subunits keep non-covalent binding in conformation before fusion. The S2 subunit also consists of a plurality of structural domains, and has a major function of mediating fusion of the virus to the host cell. There are four different structural domains of the distal S1 subunit in structure: NTD, RBD, CTD1 and CTD2, where RBD is a receptor binding structural domain, and is mainly responsible for binding to a receptor angiotensin converting enzyme 2 (ACE2) on the surface of the host cell, thereby mediating the virus to infect the host cell. Therefore, the S protein and the RBD are both major targets for current research and development of genetic engineering vaccines.

The term "trimer form" is a type in a quaternary structure of a protein. The quaternary structure of the protein refers to numbers and arrangements of protein subunits relative to each other (Chou, Kuo-Chen; Cai, Yu-Dong. Predicting protein quaternary structure by pseudo amino acid composition. Proteins: Structure, Function, and Bioinformatics. 1 November 2003, 53 (2): 282-289.). Among them, the protein including three protein subunits is in the trimer form.

The term "primary structure" is a linear sequence of an amino acid in a peptide or the protein. Conventionally, the primary structure of the protein is reported as being from an amino terminal (N terminal) to a carboxy terminal (C terminal).

The term "fusion protein" refers to one, two or more expression products obtained by DNA recombination technology after genetic recombination. A fusion protein technology is a targeted gene fusion and protein expression method performed for obtaining a great quantity of standard fusion proteins. A novel target protein with a plurality of functions may be constructed and expressed using the fusion protein technology.

The term "vector" is a nucleic acid vehicle, into which polynucleotide may be inserted. If the vector can make the protein, into which polynucleotide is inserted, be expressed, the vector is called an expression vector. The vector can make its carrying genetic material element be expressed in the host cell by being transformed, transduced or transfected into the host cell. The vector is well known to those skilled in the art, and includes, but is not limited to: a plasmid, phagemid, a cosmid, an artificial chromosome (for example, a yeast artificial chromosome (YAC), a bacterial artificial chromosome (BAC) or a P1-derived artificial chromosome (PAC)), a bacteriophage (such as a gamma bacteriophage, or an M13 bacteriophage), an animal virus and the like. The animal virus which may be used as the vector includes, but is not limited to: a retrovirus (including a lentivirus), an adenovirus, an adeno-associated virus, a herpes virus (such as a herpes simplex virus), a poxvirus, baculovirus, a papillomavirus, a papovavirus (such as SV40). The vector may includes a plurality of elements for controlling expression, and includes, but is not limited to: a promoter sequence, a transcription initiation sequence, an enhancer sequence, a selection element and a reporter gene. In addition, the vector may further includes a replication origin.

The term "host cell" is a cell, into which the nucleic acid molecule has been introduced through a molecular biological technology. These technologies include transfection of the viral vector, transformation with the plasmid vector, and introduction of naked DNA through electroporation, lipofection and particle gun acceleration.

The term "treatment" refers to that the possibility of disease pathologies and occurrence of disease symptoms are reduced, so that, for example, a subject has a longer survival time or reduced discomfort to a certain degree. Treatment may refer to the ability of a therapy reducing the disease symptoms, signs or causes if a therapy is given to the subject. Treatment further refers to relieving or reducing at least on clinical symptom, and/or inhibiting or delaying a progress of a symptom, and/or preventing or delaying the onset of a disease or a condition.

The term "subject" refers to any person or other animals, particularly, other mammals, receiving prevention, treatment and diagnosis. Other mammals may include, for example, a dog, a cat, cattle, horse, sheep, pig, a goat, a rabbit, a rat, a guinea pig, a mouse and the like.

To make those skilled in the art better understand the technical solutions of the present invention, the present invention will be further described in detail below in combination with the specific examples.

### Example 1: Design Of S-RBD Trimer Protein For Novel Coronavirus Based On Structural Biology

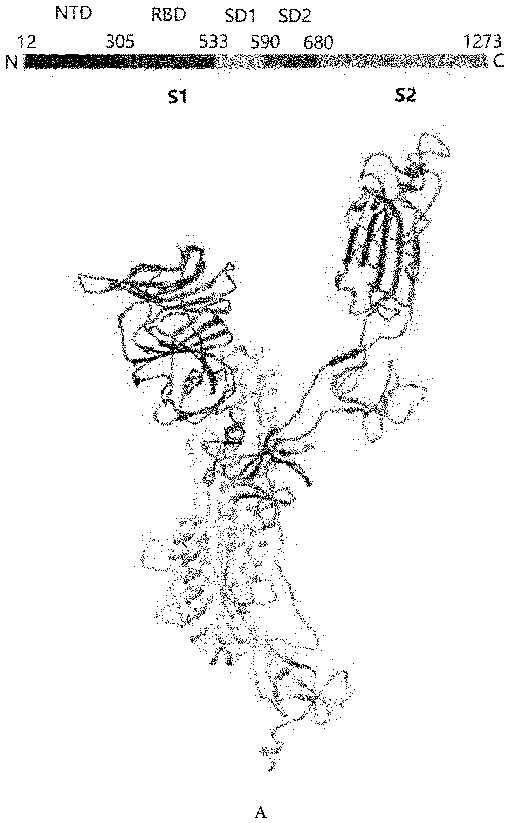

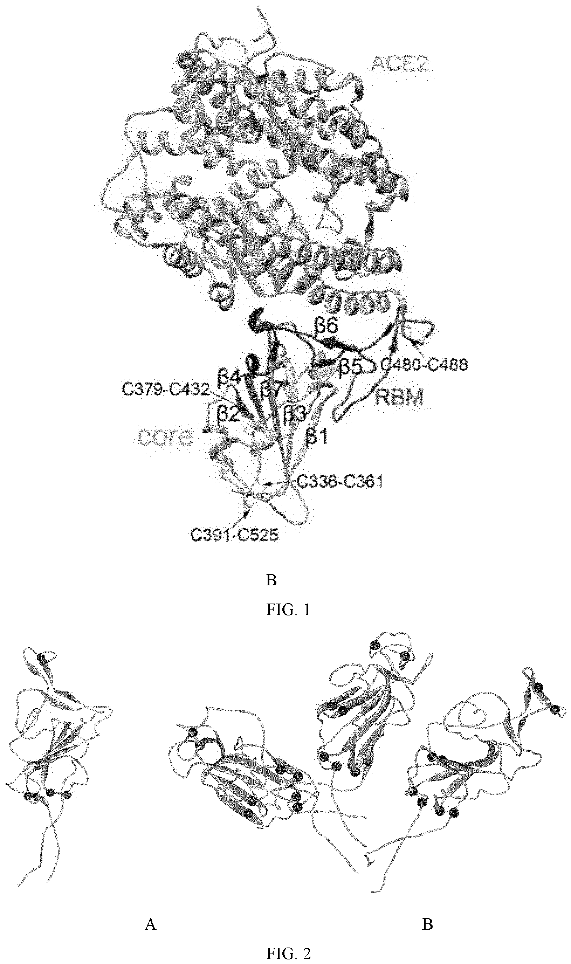

Through analysis on a space structure of a natural S protein trimer (as shown in FIG. 1) and calculation on a spatial distance between an N terminal and a C terminal of an RBD structural domain, it is discovered that in the case without introducing an exogenous vector or a sequence, the own structural features of the RBD may be used for achieving trimerization; there will be no large steric hindrance at the same time; and a receptor binding site and a main neutralizing antibody epitope cannot be covered either. Based on this, sequence fragments (at positions 319-537) in the RBD domain of an S protein of the novel coronavirus (with a Genebank number being MN908947.3) were intercepted with an interval between the N terminal and the C terminal being 14Å; and three same fragments in the RBD domain were linked end to end, so as to form a new fusion protein with an amino acid sequence shown as SEQ ID NO. 1. By using a polymer structure prediction software (initio domain assembly, AIDA) and molecular dynamics simulation (GROMACS), it shows that the fusion protein includes three independent RBD structural domains to form a trimer form with stable antigen conformation (as shown in FIG. 2), which is the S-RBD trimer protein for the novel coronavirus.

### Example 2: Expression, Purification And Identification Of S-RBD Trimer Protein For Novel Coronavirus

According to codon preference of a CHO cell expression system, codon optimization was performed on the amino acid sequence shown as SEQ ID NO. 1, and the optimized gene sequence is shown as SEQ ID NO. 2; after an expression vector for a CHO cell was constructed, the expression vector for the CHO cell was transfected into a 293FT cell or the CHO cell for construction of a recombinant cell line; and a cell line capable of stable secretory expression of the S-RBD trimer protein could be obtained through screening with a limited dilution method.

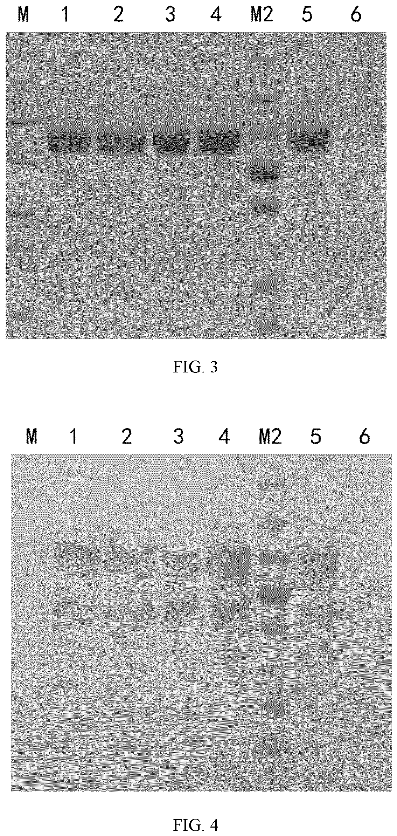

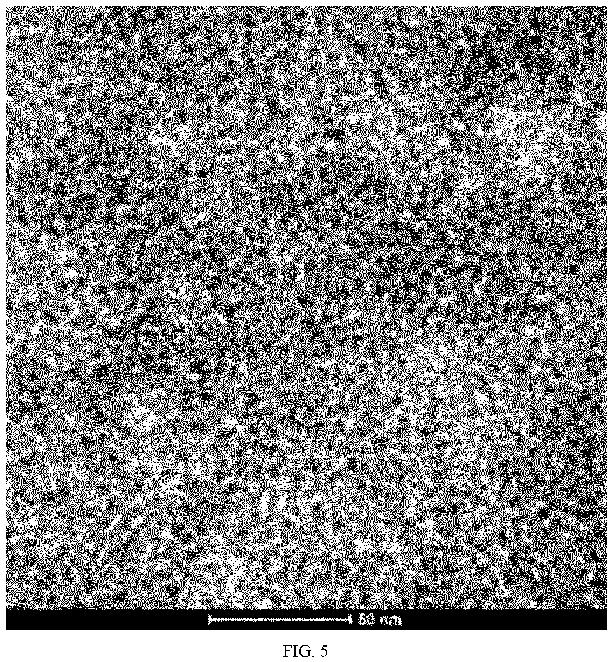

Through a series of chromatographic purification, the S-RBD trimer protein with a purity larger than or equal to 95% was obtained. An SDS-PAG detection result is shown in FIG. 3. A protein molecular weight is about 86 kD; and a part of substances (such as a dimer protein and a monomer protein) related to the product is visible at the same time. The purified protein was electrotransferred onto a polyvinylidene fluoride (PVDF) membrane after the protein was subjected to SDS-PAGE electrophoresis, and then was subjected to Western-blot identification using an RBD-specific antibody (manufacturer: Sino Biological Inc.; article number: 40591-T62; dilutability: 2000 times) (a result is shown in FIG. 4). It shows that the purified protein may bind to the RBD-specific antibody, and has good immunogenicity. The purified protein was added on a copper mesh dropwise for adsorption for 1 min and negatively stained with phosphotungstic acid for 1 min. A morphology of the protein is observed under a transmission electron microscope (a result is shown in FIG. 5); and it can be seen that the S-RBD trimer protein is uniformly distributed on the copper mesh with a small particle size.

### Example 3: Biological Analysis On Binding Of S-RBD Trimer Protein For Novel Coronavirus And Neutralizing Monoclonal Antibody

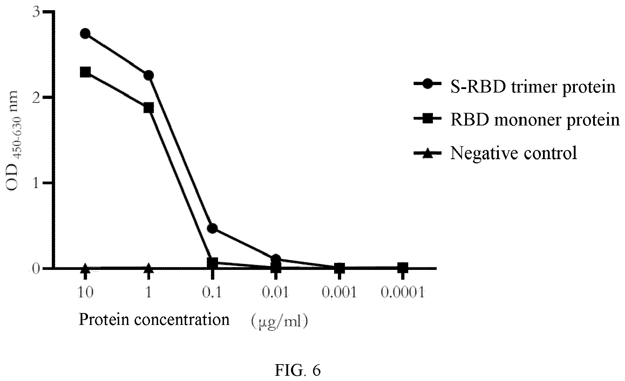

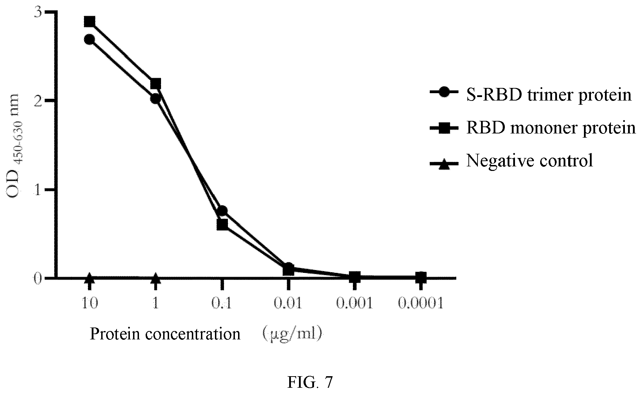

The purified S-RBD trimer proteins and a RBD monomer proteins (manufacturer: Sino Biological Inc.; article number: 40592-V08B) were diluted to 10 µg/ml, 1 µg/ml, 0.1 µg/ml, 0.01 µg/ml, 0.001 µg/ml and 0.0001 µg/ml and were used to coat a 96-well microplate by 100 µl/well at 4°C for 8-12 h, where a blank hole serves as a negative control; after the plate was washed with PBST, and a confining liquid was added for confining at 37°C for 3 h; after the plate was washed with the PBST, 100 µl/well of diluted MM43 monoclonal antibody (manufacturer: Sino Biological Inc.; article number: 40591-MM43; dilutability: 2000 times) or MM57 monoclonal antibody (manufacturer: Sino Biological Inc.; article number: 40592-MM57; dilutability: 2000 times) was added respectively for incubation at 37°C for 1 h; after the plate was washed with the PBST, 100 µl/well of diluted HEP-labeled goat anti-mouse IgG (manufacturer: Beijing Zhongshan Jinqiao Biotechnology Co. , Ltd.; article number: ZB-2305; dilutability: 10000 times) was added for incubation at 37°C for 1 h; after the plate was washed with the PBST, chromogenic solutions (manufacturer: Beijing Wantai Biological pharmaceutical Co., LTD.) A and B were added sequentially for developing a color for 5-10 min, and a stop solution C was added; values were read from a microplate reader at double wavelengths (OD450 nm and 630 nm), to determine a cutoff value, and a curve of protein concentration versus absorbance value was drawn. Results are shown in FIG. 6 and FIG. 7, and it can be seen that the S-RBD trimer protein has good binding activity to both the MM43 neutralizing monoclonal antibody and the MM57 neutralizing monoclonal antibody.

### Example 4: Biological Analysis On Binding Of S-RBD Trimer Protein For Novel Coronavirus And ACE2 Receptor

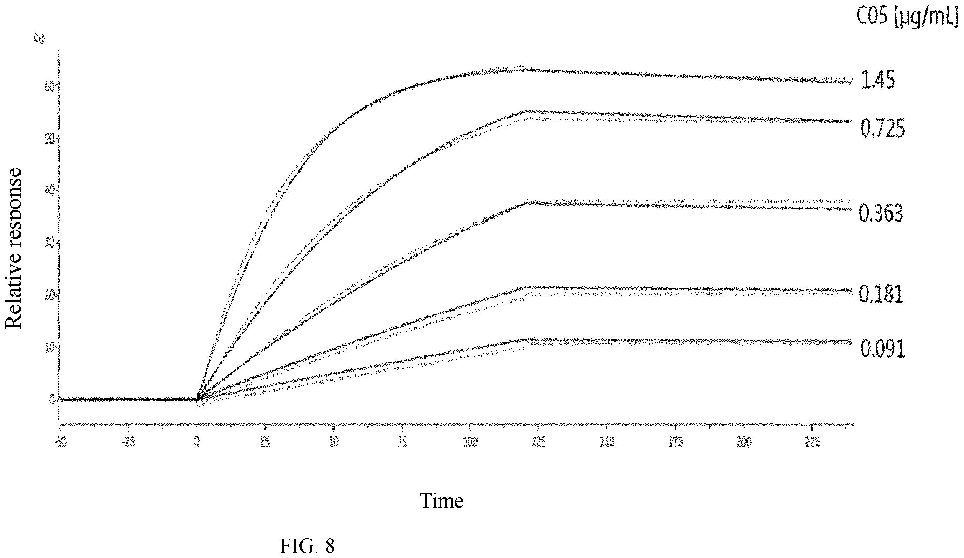

With a deep research on a structure and an infection mechanism of the novel coronavirus, more and more researchers have discovered that an angiotensin converting enzyme (ACE2) is a receptor for the novel coronavirus. Therefore, through determination on the binding condition of the S-RBD trimer protein and ACE2, the binding condition may be used for analyzing the biological activity of the protein. The affinity of the purified S-RBD trimer protein to the ACE2 protein (manufacturer: Sino Biological Inc.; article number: 10108-H08B) is determined using surface plasmon resonance (SPR). A result is shown in FIG. 8 that an affinity constant (kD) value is 3.90 e-12M; and the S-RBD trimer protein presents high affinity to bind to the ACE2, which prompts that the protein has good biological activity.

### Example 5: Preparation Of Recombinant Novel Coronavirus Vaccine

The purified S-RBD trimer protein was diluted to twice a target antigen concentration and was mixed with 1.2 mg/ml adjuvant (i.e. aluminium hydroxide) in a ratio of 1: 1 (w/w) for adsorption; a mixture was stirred by a magnetic stirrer for 40-120 min at a rotating speed of 200-300 rpm, to obtain a semi-finished vaccine, where a content of a residual protein in a supernatant should be lower than 10% of a total protein content; and the semi-finished vaccine was aseptically dispensed into vials by 0.5 ml per vial, so as to obtain finished vaccines.

### Example 6: Evaluation On Immunological Effect Of Recombinant Novel Coronavirus Vaccine

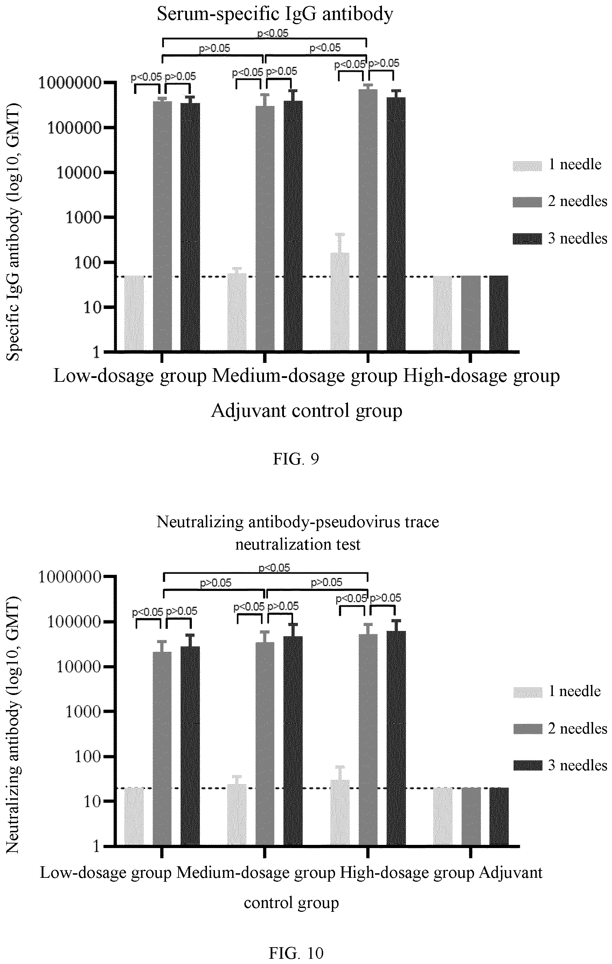

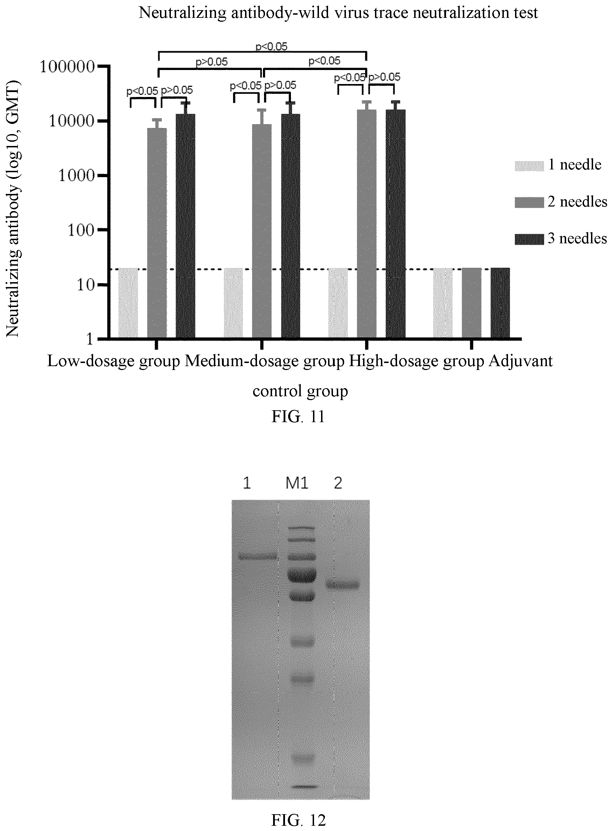

The prepared recombinant novel coronavirus vaccine was intramuscularly injected for immunizing Wistar rats (purchased from Sipeifu (Beijing) Biotechnology Co., LTD., aged from 6 weeks to 8 weeks, with an animal certificate number: No. 110324201102113753) in a dosage of 0.5 ml per rat. A specific animal test solution is shown in Table 1. A titer of a specific IgG antibody for the S-RBD protein in a serum after immunization was detected with an enzyme-linked immunosorbent assay (ELISA), and a titer of a neutralizing antibody was detected using pseudovirus and wild virus trace neutralization tests. A result of serum-specific IgG antibody is shown in FIG. 9; a result of the pseudovirus trace neutralization test is shown in FIG. 10; a result of the wild virus trace neutralization test is shown in FIG. 11; and GMT values of the serum antibodies are shown in Table 2. It can be seen that the recombinant novel coronavirus vaccine may stimulate the rats to generate a high-titer antibody level; and with the increase in a number of immunization needles or an immunization dosage, the levels of the specific IgG antibodies and the neutralizing antibodies are gradually increased, and there is an obvious dosage relationship.

Detection on the serum-specific IgG antibody: the level of the specific IgG antibody for the S-RBD protein in the serum after immunization was detected by using the ELISA; a commercial RBD protein (purchased from Sino Biological Inc., with an article number: 40592-V08B) was diluted to 1 µg/ml and was used to coat a microplate by 100 µl/well for confining; then, a serum subjected to a series of dilution was added; a signal was detected using an enzyme-labeled antibody (rat-derived) to determine a cut-off value; and a highest dilution fold of a positive serum is the titer of the specific IgG antibody for the serum sample.

The pseudovirus trace neutralization test: a gene sequence of the S protein in the pseudovirus was derived from a Wuhan-Hu-1 strain (with a GenBank sequence number: MN908947); a reporter gene was firefly luciferase; and in the neutralization test, a working concentration of the pseudovirus was (1-2)×104 TCID 50/ml, a serum dilution fold at an infection inhibition rate of 50% was calculated with a Reed-Muench method, and it was the titer of the neutralizing antibody for the serum sample.

The wild virus trace neutralization test: the test was finished in a BSL3-grade laboratory, and a used virus stain was a 2020XN4276 stain; a serum subjected to a series of dilution was mixed with an equal volume of a virus (100TCID50) for incubation; a mixture was neutralized in an incubator at 37°C for 2 h; a resultant was added to a cell culture plate including Vero-E6 cells (2×105/ml); a cell control and a virus control were set at the same time; the resultant was cultured in the incubator at 37°C for 3-5 d; a cytopathic condition was observed; and a serum dilution fold at the infection inhibition rate of 50% was calculated with the Reed-Muench method, and it was the titer of the neutralizing antibody for the serum sample.

**Table 1: Wistar Rat Test Solution Of Recombinant Novel Coronavirus Vaccine**

| Number | Content of antigen | Content of aluminium adjuvant | Immunization program | Blood sampling time | Number of animals |
|---|---|---|---|---|---|
| Low-dosage group | 10µg/dose | 0.30mg/do se | Immunized with 0w, 3w and 6w for 3 needles | Sampling blood by 1w after immunization per dose | 8 |
| Medium-dosage group | 20µg/dose | 0.30mg/do se | | | 8 |
| High-dosage group | 40µg/dose | 0.30mg/do se | | | 8 |
| Adjuvant control | None | 0.30mg/do se | | | 8 |

**Table 2: GMT Values of Serum of Rats Immunized With Recombinant Novel Coronavirus Vaccine**

| Number | Content of antigen | GMT values of serum | | | | | | | | |
|---|---|---|---|---|---|---|---|---|---|---|
| | | Serum-specific IgG antibody | | | Neutralizing antibody (pseudovirus) | | | Neutralizing antibody (wild virus) | | |
| | | 1 | 2 | 3 | 1 | 2 | 3 | 1 | 2 | 3 |
| Low-dosage group | 10µg/dose | 50 | 366802 | 320000 | 20 | 15604 | 21880 | 20 | 6640 | 11167 |
| Medium-dosage group | 20µg/dose | 55 | 237841 | 293441 | 23 | 25874 | 32580 | 20 | 6640 | 11167 |
| High-dosage group | 40µg/dose | 92 | 672717 | 414989 | 24 | 43185 | 50465 | 20 | 14482 | 14482 |
| Adjuvant control | None | 50 | 50 | 50 | 20 | 20 | 20 | 20 | 20 | 20 |

### Comparative Example 1: Comparasion On S-RBD Trimer Protein And Dimer Protein

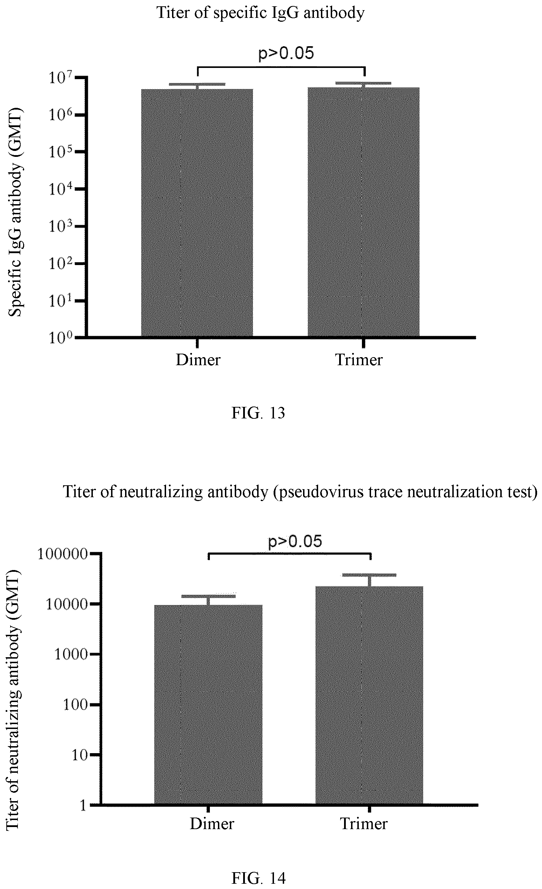

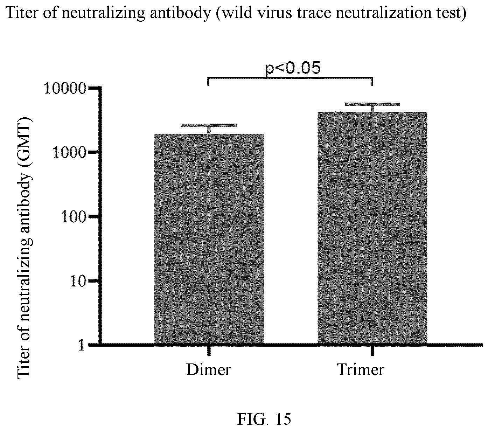

Sequence fragments (at positions 319-537) in an RBD domain of an S protein of the novel coronavirus (with a Genebank number being MN908947.3) were intercepted, and two same fragments in the RBD domain were linked end to end, so as to form a dimer fusion protein with an amino acid sequence shown as SEQ ID NO. 3. According to codon preference of a CHO cell expression system, the amino acid sequence shown as SEQ ID NO. 3 was performed codon optimization, and the optimized gene sequence is shown as SEQ ID NO. 4; and after an expression vector for a CHO cell was constructed, the expression vector for the CHO cell was transfected into a 293FT cell to obtain a cell line capable of expressing the dimer protein. Through a series of chromatographic purification, the S-RBD dimer protein with a purity larger than or equal to 95% was obtained (as shown in FIG. 12). The S-RBD dimer protein was mixed with 1.2 mg/ml adjuvant (i.e. aluminium hydroxide) in a ratio of 1: 1 (w/w) for adsorption to prepare a dimer protein immune material; the dimer protein immune material and a trimer protein immune material prepared with the same process were intraperitoneally injected to immunize BALB/c mice (with a content of the antigen being 2.0 µg/dose and a content of an aluminium adjuvant being 0.30 mg/dose) for 3 needles by 0w, 1w and 2w; serums were collected and separated by 2w after immunization in the whole process; a titer of the specific IgG antibody for the S-RBD protein in each serum after immunization was detected with the ELISA; and titers of the neutralizing antibodies were detected with pseudovirus and wild virus trace neutralization tests. Detection results are shown in FIGs. 13-15; and GMT detection results are shown in Table 3. It can be seen that the levels of the antibodies produced by the trimer protein are all higher than those produced by the dimer protein, where there is a statistic difference in titer of the specific IgG antibodies; although the titer of the neutralizing antibody for the pseudovirus is higher by about 2 times, there is no statistic difference; and although the titer of the neutralizing antibody for the wild virus is higher by about 3 times, a difference has the statistical significance.

**Table 3 GMT Values Of Serum Immunized With Trimer Protein And Dimer Protein**

| Type of proteins | GMT value of serum antibody | | |
|---|---|---|---|
| | Specific IgG antibody | Neutralizing antibody for pseudovirus | Neutralizing antibody for wild virus |
| Dimer protein | 4525483 | 8440 | 1810 |
| Trimer protein | 5079683 | 16037 | 4064 |

The above are preferred embodiments of the present invention, and it should be noted that, for those of ordinary skill in the art, several improvements and modifications may be made without departing from the principle of the present invention, and the improvements and modifications are also regarded to be within the protection scope of the present invention.

## Claims

1. An S-RBD trimer protein for a novel coronavirus, **characterized in that** the trimer protein is composed of amino acid fragments at positions 319-537 in an RBD domain of an S protein of the novel coronavirus in a trimer form.

2. The S-RBD trimer protein for the novel coronavirus according to claim 1, **characterized in that** a primary structure of the trimer protein is formed by linking three of the amino acid fragments in an order from an N terminal to a C terminal.

3. The S-RBD trimer protein for the novel coronavirus according to claim 2, **characterized in that** the S-RBD trimer protein for the novel coronavirus comprises an amino acid sequence shown as SEQ ID No. 1 or a sequence having a homology of 95% or higher with SEQ ID No. 1.

4. A fusion protein, comprising the S-RBD trimer protein for the novel coronavirus according to claim 1.

5. The fusion protein according to claim 4, the fusion protein further comprising one or more of a signal peptide, a tag or an immune-enhancing peptide.

6. A nucleic acid molecule, comprising a nucleotide sequence encoding the S-RBD trimer protein for the novel coronavirus according to claim 1.

7. A nucleic acid molecule, comprising a nucleotide sequence encoding the fusion protein according to claim 4.

8. The nucleic acid molecule according to claim 6, **characterized in that** the nucleic acid molecule comprises an nucleotide sequence shown as SEQ ID No. 2 or a sequence having a homology of 95% or higher with SEQ ID No. 2.

9. A vector, comprising the nucleic acid molecule according to claim 6.

10. A vector, comprising the nucleic acid molecule according to claim 7.

11. A host cell, comprising the nucleic acid molecule according to claim 6.

12. A host cell, comprising the vector according to claim 9.

13. A host cell, comprising the vector according to claim 10.

14. The host cell according to claim 11, **characterized in that** the host cell is *Escherichia coli*, a yeast cell, an insect cell or a mammalian cell.

15. The host cell according to claim 13, **characterized in that** the host cell is a Chinese hamster ovary (CHO) cell.

16. A preparation method for the S-RBD trimer protein for the novel coronavirus according to claim 1, the mathod comprising the following steps:
step A): preparing the nucleic acid molecule according to claim 6, constructing the expression vector according to claim 9, and transforming or transfecting the expression vector into the host cell according to claim 12;
step B): performing, by using a product in step A), protein expression; and
step C): obtaining, by purifying an expression product obtained in step B), the S-RBD trimer protein for the novel coronavirus.

17. The preparation method for the fusion protein according to claim 4, the method further comprising the following steps:
step A): preparing the nucleic acid molecule according to claim 7, constructing the expression vector according to claim 10, and transforming or transfecting the expression vector into the host cell according to claim 13;
step B): performing, by using a product in step A), protein expression; and
step C): obtaining, by purifying an expression product obtained in step B), the fusion protein.

18. A use of the S-RBD trimer protein for the novel coronavirus according to claim 1 in preparing a drug for treating and/or preparing novel coronavirus infection and/or a disease caused by the novel coronavirus.

19. A use of the fusion protein according to claim 4 in preparing a drug for treating and/or preparing novel coronavirus infection and/or a disease caused by the novel coronavirus.

20. A use of the nucleic acid molecule according to claim 6 in preparing a drug for treating and/or preparing novel coronavirus infection and/or a disease caused by the novel coronavirus.

21. A use of the vector according to claim 9 in preparing a drug for treating and/or preparing novel coronavirus infection and/or a disease caused by the novel coronavirus.

22. A use of the host cell according to claim 11 in preparing a drug for treating and/or preparing novel coronavirus infection and/or a disease caused by the novel coronavirus.

23. A vaccine, comprising the S-RBD trimer protein for the novel coronavirus according to claim 1 and an adjuvant.

24. A vaccine, comprising the fusion protein according to claim 4 and an adjuvant.

25. The vaccine according to claim 23, **characterized in that** the adjuvant is aluminium hydroxide, aluminium phosphate, MF59 or CpG.

26. The vaccine according to claim 25, **characterized in that** the adjuvant is the aluminium hydroxide.

27. A preparation method for the vaccine according to claim 23, **characterized in that** the S-RBD trimer protein for the novel coronavirus obtained by purification is mixed with the adjuvant.

28. A preparation method for the vaccine according to claim 24, **characterized in that** the fusion protein obtained by purification is mixed with the adjuvant.

29. A use of the vaccine according to claim 23 in treating and/or preparing novel coronavirus infection and/or a disease caused by the novel coronavirus.

30. Use of the vaccine according to claim 24 for treating and/or preventing SARS-CoV-2 infection and/or diseases caused by SARS-CoV-2.

31. A drug composition, comprising the vaccine according to claim 23 and a pharmaceutically acceptable vector.

32. A method of eliciting an immune response of a subject to the novel coronavirus or treating the novel coronavirus infection of the subject, **characterized in that** an effective dosage of the vaccine according to claim 23 is applied to the subject.

33. A method of eliciting an immune response of a subject to the novel coronavirus or treating the novel coronavirus infection of the subject, **characterized in that** an effective dosage of the drug composition according to claim 32 is applied to the subject.
